Europäisches Patentamt

European Patent Office

Office européen des brevets

(11) Veröffentlichungsnummer: **0 420 034 A1**

# EUROPÄISCHE PATENTANMELDUNG

(21) Anmeldenummer: 90118078.6

(22) Anmeldetag: 20.09.90

(51) Int. Cl.5: **C07C 51/41, C10L 1/18**

(30) Priorität: 28.09.89 DE 3932322

(43) Veröffentlichungstag der Anmeldung:
03.04.91 Patentblatt 91/14

(84) Benannte Vertragsstaaten:
AT BE CH DE ES FR GB IT LI NL SE

(71) Anmelder: HOECHST AKTIENGESELLSCHAFT
Postfach 80 03 20
W-6230 Frankfurt am Main 80(DE)

(72) Erfinder: Weber, Jürgen, Dr. Dipl.-Chem.
Bunsenstrasse 17
W-4200 Oberhausen 11(DE)
Erfinder: Lappe, Peter, Dr. Dipl.-Chem.
Eickenhof 34
W-4220 Dinslaken(DE)
Erfinder: De Win, Werner, Dipl.-Ing.
Heimstättenweg 18
W-4220 Dinslaken(DE)
Erfinder: Nierhaus, Wolfgang
Hattropstrasse 42
W-4130 Moers 1(DE)

(54) Verfahren zur Herstellung von Gemischen öllöslicher Eisen- und Magnesiumsalze gesättigter aliphatischer Monocarbonsäuren und ihre Verwendung.

(57) Zur Herstellung eines Gemisches öllöslicher Eisen- und Magnesiumsalze gesättigter aliphatischer $C_6$- bis $C_8$-Monocarbonsäuren wird Magnesiumoxid in einer Lösung eines Eisensalzes einer gesättigten $C_6$- bis $C_8$-Monocarbonsäure mit einer $C_6$- bis $C_8$ Monocarbonsäure umgesetzt. Das Gemisch findet als Verbrennungshilfsmittel Verwendung.

EP 0 420 034 A1

## VERFAHREN ZUR HERSTELLUNG VON GEMISCHEN ÖLLÖSLICHER EISEN- UND MAGNESIUMSALZE GESÄTTIGTER ALIPHATISCHER MONOCARBONSÄUREN UND IHRE VERWENDUNG

Die Erfindung betrifft ein Verfahren zur Herstellung von Gemischen aus Eisen- und Magnesiumsalzen, gesättigter aliphatischer Monocarbonsäuren mit 6 bis 8 Kohlenstoffatomen und die Verwendung dieser Gemische als Verbrennungshilfsmittel für flüssige Brennstoffe.

Die durch Verknappung und Verteuerung ausgelöste sparsamere Verwendung von Erdöl führte u.a. zur Entwicklung von Verfahren, die eine möglichst optimale Nutzung dieses Rohstoffes gewährleisten. Ein beträchtlicher Anteil des Erdöls wird zu schwerem und leichtem Heizöl umgearbeitet und zur Energieerzeugung, insbesondere zur Wärme- und Elektrizitätsgewinnung eingesetzt. Der hierbei erzielbare Wirkungsgrad hängt von der Vollständigkeit der Verbrennung des Brennstoffs ab. Daher ergreift man verschiedene Maßnahmen, die eine vollständige Verbrennung, d.h. eine Vermeidung der Rußbildung bewirken.

Ein Weg dieses Ziel zu erreichen, besteht im Zusatz bestimmter Stoffe zum Heizöl, die dessen Verbrennung fördern. Als die Rußbildung unterdrückende Additive sind seit langem Sulfonate und Naphthenate verschiedener Metalle bekannt (vgl. z.B. J. Vaerman, Journal of the Institute of Petroleum, Vol. 50, Nr. 487 (1964), S. 155 -168).

Auch suspendierte anorganische Metallsalze und Metalloxide wirken stark verbrennungsfördernd (vgl. P.J. Agius et al., 8th World Petroleum Cogress Proceedings 5, 27 -33 (1971)), haben aber ähnlich den Metallchelaten und Acetylacetonaten und dem Ferrocen den Nachteil, sich leicht aus der Suspension abzuscheiden.

Weiterhin sind aus der FR-PS 13 81 150 Eisen- und Mangansalze aliphatischer Carbonsäuren mit 10 bis 30 Kohlenstoffatomen als Additive bekannt, die die Verbrennung flüssiger Brennstoffe verbessern.

Schließlich ist in der DE 30 44 907 C2 die Verwendung von Eisen- und/oder Mangansalzen aliphatischer Carbonsäuren mit 6 bis 8 Kohlenstoffatomen als Verbrenungshilfsmittel beschrieben. Diese Verbindungen haben den Vorteil gut öllöslich und ungiftig und darüber hinaus leicht zugänglich zu sein.

Durch Verwendung der vorstehend aufgeführten Additive wird bei der Verbrennung flüssiger Brennstoffe zwar die Rußbildung vollständig oder zumindest nahezu vollständig unterdrückt. Sie tragen jedoch nichts zur Lösung des Schwefelproblems bei. Schwere wie leichte Heizöle enthalten nämlich entsprechend ihrer Herkunft mehr oder weniger große Anteile gebundenen Schwefels. In der Flamme wird dieser Schwefel teilweise zu $SO_3$

verbrannt, wobei u.a. ebenfalls in den Heizölen enthaltene Vanadiumverbindungen als Katalysatoren wirken. In Gegenwart von Wasser bildet $SO_3$ korrosive Schwefelsäure. Zur Vermeidung von Schäden in den Verbrennungsanlagen müssen die Abgase daher über den Säuretaupunkt erhitzt werden um die Kondensation der Säure zu verhindern.

Obgleich durch konstruktive Maßnahmen größere Schäden infolge Korrosion ausgeschlossen werden können, beschreitet man wegen der Vielfalt der Verbraucher mit ihren verschiedenen Feuerungsanlagen in der Praxis einen anderen Weg. Es hat sich nämlich gezeigt, daß durch Zusatz von Magnesium in Form einer in den Heizölen löslichen Verbindung die Bildung von Schwefelsäure vermieden werden kann. Das bei der Verbrennung der im Öl gelösten Magnesiumverbindung gebildete Magnesiumoxid desaktiviert nicht nur die Stoffe, die die Oxidation des Schwefels zu $SO_3$ katalysieren. Es reagiert auch mit Schwefelsäure zu Magnesiumsulfat, das sich als schützender Staub auf die Anlagenteile der Verbrennungsvorrichtung legt und überdies nicht die Umwelt belastet, wenn es ins Freie gelangt.

Durch Zusatz öllöslicher Eisen- und Magnesiumverbindungen lassen sich daher bei der Verbrennung von Heizölen die Rußentstehung und die $SO_3$-Bildung drastisch verringern. Daraus resultiert eine Erhöhung der Wirtschaftlichkeit der Verwendung von Heizöl, denn der Kohlenstoffgehalt des Öls wird besser genutzt, die Temperatur der Verbrennungsgase kann gesenkt werden und freigesetzte Schwefelsäure kann weder in den Verbrennungvorrichtungen noch in der Umwelt Schäden verursachen.

Die als Additive für Heizöle verwendeten Eisen- bzw. Magnesiumsalze aliphatischer Carbonsäuren werden getrennt hergestellt. Üblicherweise geht man von einem wasserlöslichen Eisensalz z.B. dem Nitrat aus, das man mit einer aliphatischen Carbonsäure in Gegenwart von Alkalihydroxid umsetzt. Das Magnesiumsalz erhält man durch Reaktion von Magnesiumoxid oder Magnesiumcarbonat mit der Carbonsäure bei Temperaturen oberhalb 120° C. Trotz der hohen Temperaturen beträgt die Reaktionszeit mindestens 2 Stunden. Die Anwendung so hoher Temperaturen hat Verbackungen im Reaktor zur Folge, die eine gleichförmige Umsetzung von Magnesiumoxid und Carbonsäure beeinträchtigen, daher zu unreinen Produkten führen und die Entleerung des Reaktors erschweren.

Es stellte sich daher die Aufgabe eine Arbeitsweise zu entwickeln, die es erlaubt, Mischungen aus öllöslichen Eisen- und Magnesiumsalzen aliphatischer Carbonsäuren in einfacher Weise ohne Auftreten von Verbackungen und innerhalb kurzer Reaktionszeiten zu gewinnen.

Die Erfindung besteht in einem Verfahren zur Herstellung eines Gemisches öllöslicher Eisen- und Magnesiumsalze gesättigter aliphatischer Monocarbonsäuren. Es ist dadurch gekennzeichnet, daß man in einer Lösung eines Eisensalzes einer gesättigten aliphatischen Monocarbonsäure mit 6 bis 8 Kohlenstoffatomen in einem organischen Lösungsmittel Magnesiumoxid mit der äquivalenten Menge oder einem Überschuß einer gesättigten aliphatischen Monocarbonsäure mit 6 bis 8 Kohlenstoffatomen bei Temperaturen von 50 bis 100 °C umsetzt.

Überraschenderweise wird nach dem neuen Verfahren die Reaktionszeit für die Umsetzung des Magnesiumoxids mit der aliphatischen Carbonsäure deutlich gegenüber einem Prozeß verringert, bei dem allein Magnesiumoxid und die aliphatische Carbonsäure umgesetzt werden. Weder während der Reaktion noch bei der Entleerung des Reaktors treten Probleme durch Verbackung auf. Das Magnesiumoxid hat völlig durchreagiert, so daß das Carboxylat nicht durch die eingesetzte Magnesiumverbindung verunreinigt ist.

Als Reaktionsmedium verwendet man die Lösung eines Eisensalzes einer gesättigten aliphatischen Monocarbonsäure mit 6 bis 8 Kohlenstoffatomen in einem geeigneten organischen Lösungsmittel. Die dem Salz zugrundeliegende Säure kann geradkettig oder verzweigt und insbesondere α-verzweigt sein. Beispiele für solche Säuren sind 2-Ethylbuttersäure, 2,3-Dimethylbuttersäure, 2-Methylpentansäure, 2-Ethylpentansäure, 2-Ethylhexansäure und Isooctansäure. Bevorzugt ist die 2-Ethylhexansäure. Das Eisen liegt in den Salzen als Eisen(III)-ion vor.

Als organische Lösungsmittel kommen solche organischen Substanzen oder Substanzgemische in Betracht, in denen die Carboxylate, also sowohl das Eisensalz als auch das Magnesiumsalz der $C_6$- bis $C_8$-Monocarbonsäure und überdies die freie $C_6$- bis $C_8$ Monocarbonsäure löslich sind. Bewährt haben sich aliphatische oder aromatische Kohlenwasserstoffe oder Kohlenwasserstoffgemische wie Kerosin, Toluol und Xylol und insbesondere Mineralölfraktionen mit Siedegrenzen zwischen etwa 150 und etwa 300 °C.

Die Herstellung des Reaktionsmediums erfolgt durch einfaches Lösen des Eisencarboxylats in dem organischen Lösungsmittel. Man kann auch von der wäßrigen Lösung eines Eisensalzes, z.B. des Nitrats ausgehen, der man die äquivalente Menge oder einen geringen Unter- oder Überschuß eines Alkalisalzes der Monocarbonsäure, ebenfalls in wäßriger Lösung, hinzufügt. Anschließend extrahiert man die wäßrige Phase mit dem organischen Lösungsmittel. Es ist möglich, anstelle des Alkalicarboxylats die der Eisenmenge äquivalenten Mengen Monocarbonsäure und Alkalihydroxid bzw. -carbonat einzusetzen. Auch in diesem Fall schadet ein Unter- oder Überschuß von Säure und Alkaliverbindung nicht.

In der Eisencarboxylat-Lösung als Reaktionsmedium wird eine der umzusetzenden Magnesiumoxidmenge äquivalente oder überschüssige Menge einer $C_6$- bis $C_8$-Monocarbonsäure gelöst. Sie kann geradkettig oder verzweigt sein, insbesondere ist sie α-verzweigt. Beispiele für solche Säuren sind 2-Ethylbuttersäure, 2,3-Dimethylbuttersäure, 2-Methylpentansäure, 2-Ethylpentansäure, 2-Ethylhexansäure, bevorzugt wird die 2-Ethylhexansäure.

Üblicherweise wählt man die Säure, deren Rest das Anion des Eisensalzes bildet. Der Säureüberschuß soll nicht mehr als 30%, zweckmäßig etwa 10 bis 30% der äquivalenten Säuremenge betragen.

In der vorstehend beschriebenen Lösung wird das Magnesiumoxid suspendiert. Als Magnesiumoxid kann man die handelsüblichen Typen verwenden. Besonders geeignet sind schwach calcinierte Magnesiumoxide, also solche, die z.B. aus dem Carbonat durch $CO_2$-Abspaltung dicht oberhalb des Zersetzungspunktes hervorgegangen sind. Das Magnesiumoxid wird durch Erhitzen des Reaktionsgemisches auf Temperaturen zwischen 50 bis 100 °C, insbesondere 60 bis 80 °C in Lösung gebracht. Höhere Temperaturen sind wegen der Gefahr, daß Verbackungen auftreten zu vermeiden, niedrigere Temperaturen führen zu einer Verlängerung der Reaktionszeiten.

Selbstverständlich sind auch andere Varianten der Umsetzung zwischen Magnesiumoxid und Monocarbonsäure möglich. So kann man das Magnesiumoxid in dem Reaktionsmedium suspendieren und die Säure anteilsweise dem Reaktionsgemisch zusetzen.

Nach einer weiteren Ausführungsform des erfindungsgemäßen Verfahrens gibt man zu der wäßrigen Lösung eines Eisensalzes die äquivalente Menge (oder einen geringen Unter-oder Überschuß) Alkalicarboxylat und die zur Umsetzung des Magnesiumoxids erforderliche Menge der Monocarbon säure. Das Gemisch wird daraufhin mit dem organischen Lösungsmittel extrahiert. Man trennt die wäßrige Phase ab, gibt zu der organischen Phase das Magnesiumoxid und erhitzt bis das Magnesiumoxid gelöst ist. Es versteht sich von selbst, daß das Alkalicarboxylat durch Carbonsäure und die äquivalente Menge Alkalihydroxid oder -carbonat ersetzt werden kann.

Die Konzentration des Eisensalzes in der als Reaktionsmedium verwendeten organischen Lösung ist nicht kritisch. Sie hängt weitgehend von der Löslichkeit der Eisen- und der Magnesiumverbindung in dem Lösungsmittel und der weiteren Verwendung der Lösung ab.

Auch die Konzentration beider Salze in dem Lösungsmittel kann über weite Bereiche variiert werden. Entscheidend ist wiederum die vorgesehene Verwendung der Lösung und ihre Handhabbarkeit, denn mit zunehmendem Salzgehalt nimmt ihre Fließfähigkeit ab. Üblicherweise enthalten Lösungen in Mineralölfraktionen als Lösungsmittel 5 bis 8 Gew.-% der Salze, wobei als Faustregel gelten kann, daß Gemische, in denen das Eisensalz überwiegt Lösungen höherer Konzentration ergeben als Gemische mit einem größeren Anteil des Magnesiumsalzes.

Die erfindungsgemäßen Mischungen aus öllöslichen Eisen-und Magnesiumcarboxylaten haben sich als Verbrennungshilfsmittel für flüssige Brennstoffe bewährt. Unter flüssigen Brennstoffen im Sinne der vorliegenden Erfindung werden Stoffe zur Wärmeerzeugung wie Mitteldestillate des Erdöls, z.B. Heizöl EL oder schweres Heizöl verstanden. Das Salzgemisch wird dem flüssigen Brennstoff in einer solchen Menge und Zusammensetzung zugesetzt, daß die Eisenkonzentration im Brennstoff 5 bis 100 ppm beträgt und die als Salz vorliegende Magnesiummenge ausreicht, um bis zu 30 Gew.-% des im Brennstoff enthaltenen Schwefels als $MgSO_4$ zu binden. Vorzugsweise soll die Eisenkonzentration im Brennstoff 5 bis 25 ppm betragen und die Magnesiummenge ausreichen, um den in Schwefeltrioxid umgewandelten Schwefel zu binden.

Das Salzgemisch kann dem Brennstoff für sich allein oder zusammen mit anderen Additiven in fester oder gelöster Form zugesetzt werden.

Es hat sich bewährt das Salzgemisch in der bei der Herstellung erhaltenen Lösung zu verwenden.

In dem folgenden Beispiel wird das erfindungsgemäße Verfahren näher beschrieben, ohne es auf diese Ausführungsform zu beschränken.

Beispiel

Zu einer Lösung von 64,7 g Eisennitrat in 200 ml Wasser wird bei 80° C innerhalb 10 min eine Mischung aus 59,2 g Natronlauge (32,4 Gew.-%, entsprechend 0,48 mol) und 152,8 g 2-Ethylhexansäure (1,06 mol) getropft. Man läßt bei 80° C 20 min nachreagieren und fügt etwa 150 ml einer oberhalb 270° C siedenden Mineralölfraktion hinzu. Nach Abkühlen auf 40° C trennt man wäßrige und organische Phase. In die auf 80° C erhitzte organische Lösung gibt man 8,2 g (0,2 mol) Magnesiumoxid, es löst sich innerhalb 1 h vollständig auf. Die Lösung der beiden Carboxylate ist noch durch Wasser leicht getrübt; es kann durch Zentrifugieren aus der Ölphase entfernt werden. Die Lösung enthält 3 Gew.-% Eisen, 1,6 Gew.-% Magnesium, ihre Viskosität bei 20° C beträgt etwa 53 mPa s.

**Ansprüche**

1. Verfahren zur Herstellung eines Gemisches öllöslicher Eisen- und Magnesiumsalze gesättigter aliphatischer Monocarbonsäuren dadurch gekennzeichnet, daß man in einer Lösung eines Eisensalzes einer gesättigten aliphatischen Monocarbonsäure mit 6 bis 8 Kohlenstoffatomen in einem organischen Lösungsmittel Magnesiumoxid mit der äquivalenten Menge oder einem Überschuß einer gesättigten aliphatischen Monocarbonsäure mit 6 bis 8 Kohlenstoffatomen bei Temperaturen von 50 bis 100° C umsetzt.

2. Verfahren nach Anspruch 1 dadurch gekennzeichnet, daß sich das Eisensalz von einer α-verzweigten aliphatischen Monocarbonsäure ableitet.

3. Verfahren nach Anspruch 2 dadurch gekennzeichnet, daß die α-verzweigte aliphatische Monocarbonsäure die 2-Ethylhexansäure ist.

4. Verfahren nach einem oder mehreren der Ansprüche 1 bis 3 dadurch gekennzeichnet, daß als organische Lösungmittel aliphatische oder aromatische Kohlenwasserstoffe oder Kohlenwasserstoffgemische, insbesondere Mineralölfraktionen mit Siedegrenzen zwischen etwa 150 und etwa 300° C verwendet werden.

5. Verfahren nach einem oder mehreren der Ansprüche 1 bis 4 dadurch gekennzeichnet, daß man das Magnesiumoxid mit einer α-verzweigten aliphatischen Monocarbonsäure umsetzt.

6. Verfahren nach Anspruch 5 dadurch gekennzeichnet, daß die α-verzweigte aliphatische Monocarbonsäure die 2-Ethylhexansäure ist.

7. Verfahren nach einem oder mehreren der Ansprüche 1 bis 6 dadurch gekennzeichnet, daß das Magnesiumoxid schwach calciniert ist.

8. Verfahren nach einem oder mehreren der Ansprüche 1 bis 7 dadurch gekennzeichnet, daß man das Magnesiumoxid mit der aliphatischen Monocarbonsäure bei 50 bis 100° C umsetzt.

9. Verwendung des Gemisches öllöslicher Eisen-und Magnesiumsalze gesättigter aliphatischer Monocarbonsäuren nach einem oder mehreren der Ansprüche 1 bis 8 als Verbrennungshilfsmittel für flüssige Brennstoffe.

Europäisches
Patentamt

EUROPÄISCHER
RECHERCHENBERICHT

Nummer der Anmeldung

EP 90 11 8078

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 188 115 (MOONEY) <br> * Zusammenfassung; Seite 1; Seite 5, Zeile 1; Seite 7, Zeilen 4-19; Seite 9, Zeilen 5-9; Seite 12, Zeile 13 - Seite 13, Zeile 6; Seite 14, Zeilen 5-14 * | 1-6,8,9 | C 07 C 51/41 <br> C 10 L 1/18 |
| Y | EP-A-0 058 330 (ELF) <br> * Ganzes Dokument * | 1-6,8,9 | |
| A | DE-A-3 729 930 (MÜLLER) <br> * Patentansprüche; Seite 3, Zeilen 30-40 * | 1-9 | |
| A | US-A-3 446 749 (WEISFELD et al.) <br> * Insgesamt * | 1-9 | |
| D,A | DE-A-3 044 907 (RUHRCHEMIE) <br> * Insgesamt * | 1-9 | |
| A | US-A-3 501 279 (ALLEN et al.) <br> * Insgesamt * | 1-9 | |
| A | DE-B-1 252 684 (CHEMISCHE WERKE) <br> * Insgesamt * | 1,5 | RECHERCHIERTE SACHGEBIETE (Int. Cl.5) |
| A | FR-A-1 271 037 (GHENT) <br> * Insgesamt * | 1-9 | C 10 L <br> C 07 C <br> C 10 M |
| A | FR-A-1 325 217 (CARBORUNDUM) <br> * Insgesamt * | 1-9 | |

Der vorliegende Recherchenbericht wurde für alle Patentansprüche erstellt

| Recherchenort | Abschlußdatum der Recherche | Prüfer |
|---|---|---|
| Den Haag | 07 Dezember 90 | DE LA MORINERIE B.M. |